(19)

Europäisches
Patentamt

European
Patent Office

Office européen
des brevets

(11)     **EP 3 771 468 A1**

(12)                          **EUROPEAN PATENT APPLICATION**

(43) Date of publication:
    **03.02.2021  Bulletin 2021/05**

(51) Int Cl.:
    *A61K 39/395* (2006.01)    *C12Q 1/6804* (2018.01)
    *C12Q 1/6862* (2018.01)

(21) Application number: **19189415.3**

(22) Date of filing: **31.07.2019**

(84) Designated Contracting States:
    **AL AT BE BG CH CY CZ DE DK EE ES FI FR GB**
    **GR HR HU IE IS IT LI LT LU LV MC MK MT NL NO**
    **PL PT RO RS SE SI SK SM TR**
    Designated Extension States:
    **BA ME**
    Designated Validation States:
    **KH MA MD TN**

(71) Applicants:
    • **Universitätsklinikum Hamburg-Eppendorf**
      **20246 Hamburg (DE)**
    • **Leibniz-Institut für Naturstoff-Forschung und**
      **Infektionsbiologie**
      **07745 Jena (DE)**

(72) Inventors:
    • **Wiech, Thorsten**
      **20255 Hamburg (DE)**
    • **Zipfel, Peter**
      **07743 Jena (DE)**
    • **Person, Fermin**
      **79539 Lörrach (DE)**
    • **Wulf, Sonia**
      **22529 Hamburg (DE)**

(74) Representative: **Maiwald Patent- und**
    **Rechtsanwaltsgesellschaft mbH**
    **Elisenhof**
    **Elisenstraße 3**
    **80335 München (DE)**

(54)   **C3/C5 CONVERTASE ASSAYS**

(57)    The present invention concerns a method for classifying a disorder associated with the complement system comprising the detection of the presence of C3/C5 convertase complexes. Moreover the invention refers to a method for determining whether a patient will be responsive to a modulator of the complements system, a method for determining the disorder course of a patient with a disorder associated with the complement system and a method for determining whether a candidate compound is suitable for the treatment of a disorder if the complement system and the respective kits.

**Description**

**FIELD OF THE INVENTION**

The present invention concerns a method for classifying a disorder associated with the complement system comprising the detection of the presence of C3/C5 convertase complexes. Moreover, the invention refers to a method for determining whether a patient will be responsive to a modulator of the complements system, a method for determining the disorder course of a patient with a disorder associated with the complement system and a method for determining whether a candidate compound is suitable for the treatment of a disorder of the complement system and the respective kits.

**BACKGROUND OF THE INVENTION**

There is growing evidence that deregulated complement activation contributes to or drives the pathogenesis of various diseases. Thus, targeting complement is a therapeutic option (Thurman et al., 2015; Zipfel et al, 2015). C5 convertase inhibition is an established therapy in atypical hemolytic uremic syndrome and several novel drugs target other components of the complement cascade (Reddy et al., 2017, Ricklin et al. 2018). Currently, the diagnosis of complement activation in kidney diseases is primarily based on immunohistochemical detection of deposited complement activation products in tissue, the detection of consumption of complement components in plasma or body fluids and the generation of activation fragments or cleavage products.

The activation of the complement system occurs by three different pathways; the classical/lectin pathway and the alternate pathway. The diseases of the complement system differ in their prognosis and therapy. Classifying, i.e. discriminating the various diseases and subgroups of diseases would be advantageous in order to get more insights into the pathogenesis, provide better diagnosis and more specific and efficient treatment strategies, and thus to achieve better prognosis for the specific diseases. Moreover, assays for testing potential drug candidates for the diseases associated with the deregulated complement system are needed.

Complement activation comprises three different pathways, the classical, the lectin and the alternative pathway.

In the classical pathway and the lectin pathway, the C3 convertase consisting of C4b and C2b cleaves C3 to C3b and C3a. In this classical pathway, downstream thereof, the C5 convertase consisting of C4b, C2b and C3b cleaves C5 to C5b and C5a.

In the alternative pathway, the cleavage of C3 to C3b and C3a is catalyzed by the C3 convertase consisting of C3b and Bb and subsequently the cleavage of C5 to C5b and C5a is promoted by the C5 convertase consisting of two C3b subunits and Bb.

Up to now, determining whether a disease is caused or aggravated by aberrations in the classical/lectin and/or alternative pathway has been challenging and often impossible. Moreover, a method to identify, localize and differentiate complement convertases directly in tissues and other biological specimen has been lacking.

**OBJECTIVES AND SUMMARY OF THE INVENTION**

It is an objective of the invention to establish a method to detect molecular complexes of the complement activation pathway or to discriminate between aberration of the classical/lectin and alternative pathways. More specifically, a method to discriminate assembled C3/C5 convertases of the classical/lectin and alternative pathways is provided. Close proximity of C2b and C4b is used to identify assembled C3/C5 convertase of the classical/lectin pathway and close proximity of C3b and Bb for measuring the C3/C5 convertase of the alternative pathway. This allows the diagnosis, assessment of disease course/activity and/or treatment efficacy of disorders associated the complement system.

In general, the invention relates to a method for classifying a disorder associated with the complement system, comprising the following steps:

(a) Providing a sample of a subject
(b) Detecting complexes of the complement activation pathway in the sample of the subject;
(c) Classifying the disorder associated with the complement system based on the result of step (b).

More particular, the method for classifying a disorder associated with the complement system, comprising the following steps:

(a) providing a sample of a subject
(b) detecting the presence of C3/C5 convertase complex in the sample of the subject;
(c) classifying the disorder associated with the complement system based on the result of step (b).

In particular step b) comprises the following steps:

(i) Determining the amount of C4b/C2b complexes in the sample and/or
(ii) Determining the amount of C3b/Bb complexes in the sample;

Since the C3 convertase and the C5 convertase of the classical and the lectin pathways comprise the complex of C4b and C2b, the determination of the interaction of C4b and C2b allows the specific detection of the C3/C5 convertase of the classical/lectin pathway.

By the accurate measurement of the C3/C5 convertase specific for the classical/lectin pathway, the invention thus provide for the first time an assay that allows to discriminate whether a specific disorder of the complement system is due to increased activity of the classical/lectin pathway, in particular due to the increased number of C3/C5 convertase complex of the classical/lectin pathway or upstream events that lead to an increased number of C3/C5 convertase complexes of the classical/lectin pathway.

Correspondingly, the C3 convertase and the C5 convertase of the alternative pathway comprise the complex of C3b and Bb, the determination of the interaction of C3b and Bb allows the specific detection of the C3/C5 convertase of the alternative pathway.

By the accurate measurement of the C3/C5 convertase specific for the alternative pathway, the invention thus provide for the first time an assay that allows to directly determine whether a specific disease like a disorder of the complement system is due to aberrations of the alternative pathway, in particular due to the increased activity of the C3/C5 convertase complex of the alternative pathway or upstream events that lead to an increased number of C3/C5 convertase complexes of the alternative pathway.

Thus, the method of the invention is able to identify the convertases complexes in tissue and biological specimen and be used to discriminate between a disorder associated with an aberration of the classical/lectin pathway and a disorder associated with an aberration of the alternative pathway.

The combined determination of the interaction of (i) C4b with C2b and (ii) C3b with Bb allows a particularly precise determination whether a disease associated with the complement system is due to aberrations of the classical/lectin pathway or the alternative pathway.

Typically, an increased amount of C4b/C2b complexes compared to a control is indicative for a disorder associated with the classical/lectin pathway. In some embodiments, the increased amount of C4b/C2b complexes compared to a predetermined threshold may be indicative for a disorder associated with the classical/lectin pathway.

Correspondingly, the increased amount of C3b/Bb complexes compared to a control or is indicative for a disorder associated with the alternative pathway. In some embodiments, the increased amount of C3b/Bb complexes above a predetermined threshold may be indicative for a disorder associated with the alternative pathway.

In specific embodiments, a value of the amount of C4b/C2b complexes divided by the amount of C3b/Bb complexes larger than 1 may be indicative for a disorder associated with an aberration of the classical/lectin pathway. Correspondingly, the value of the amount of C3b/Bb complexes divided by the amount of C4b/C2b complexes is larger than 1 may be indicative for a disorder associated with an aberration of the alternative pathway.

Alternatively, an increased value of the amount of C4b/C2b complexes divided by the amount of C3b/Bb complexes compared to a control may be indicative for a disorder associated with an aberration of the classical/lectin pathway. Correspondingly, an increased value of the amount of C3b/Bb complexes divided by the amount of C4b/C2b complexes compared to a control may be indicative for a disorder associated with an aberration of the alternative pathway.

Exemplarily, in step (b) the detection of the presence of the C3/C5 convertase complex may be determined by *in situ* proximity ligation in the sample. Alternatively, in step (b) the detection of the presence of the C3/C5 convertase complex is carried out by fluorescence resonance energy transfer (FRET).

Accordingly, typically in step (b) the detection of the presence of the C3/C5 convertase complex is carried out by light microscopy, such as bright field microscopy or fluorescence microscopy.

In some embodiments measurement may be carried out in the biopsy sample. Thereby an assessment step for identifying regions that are useful for measurement can be avoided.

Alternatively, measurement is carried out in intact glomeruli which may lead to highly accurate results in some cases, especially in glomerulonephritis.

Typically, the disorder associated with the complement system is selected from the group consisting of kidney disease, cancer, autoimmunity, infection, degeneration and neuropathy. Examples of kidney diseases are systemic lupus erythematodes (SLE), thrombotic microangiopathy caused by atypical hemolytic uremic syndrome (aHUS), Shiga-toxin producing *E. coli* hemolytic uremic syndrome (STEC-HUS), secondary hemolytic uremic syndrome (secondary HUS), C3 glomerulopathy, C3 glomerulonephritis, dense deposit disease, post-/parainfectious glomerulonephritis, IgA nephropathy, cryoglobulinemia, and membranoproliferative glomerulonephritis (MPGN).

In some embodiments, the disorder associated with an aberration of the classical/lectin pathway is selected from the group consisting of cryoglobulinemia, membranous nephropathy and SLE, preferably SLE.

In some embodiments, the disorder associated with an aberration of the alternative pathway is aHUS, post-/parainfectious glomerulonephritis and C3 glomerulopathy.

The sample may be selected from the group consisting of body fluid or tissue sample.

Examples of body fluids are urine, liquor, blood, plasma, serum, effusion and synovial fluid.

Preferably, the tissue sample is a kidney tissue sample, more preferably a kidney biopsy.

Another aspect of the invention refers to a method for determining whether a patient with a disorder associated with the complement system will be responsive to a modulator of the classical/lectin pathway or the alternative pathway, comprising the following steps:

(a) providing a sample of the patient;
(b) detecting the presence of C3/C5 convertase complex comprising the steps:

(i) determining the amount of C4b/C2b complexes in the sample and/or
(ii) determining the amount of C3b/Bb complexes in the sample;

(c) predicting a whether the patient will be responsive to the inhibitor of the classical/lectin pathway or alternative pathway based on the result of step (b).

Preferably, the modulator is an inhibitor.

In some embodiments, the increased amount of C4b/C2b complexes indicates that the patient will be responsive to an inhibitor of the classical/lectin pathway and/or the increased amount of C3b/Bb complexes indicates that the patient will be responsive to an inhibitor of the alternative pathway or alternatively any complement targeting inhibitor, like also the terminal complement pathway.

Another aspect of the invention refers to a method for determining the disorder course of a patient with a disorder associated with the complement system, comprising the following steps:

(a) Providing samples of a subject of at least two time points of the disease course;
(b) Detecting the presence of C3/C5 convertase complex in the samples comprising the steps:

(i) Determining the amount of C4b/C2b complexes in the samples and/or
(ii) Determining the amount of C3b/Bb complexes in the samples;

(c) Determining the disorder course based on the result of step (b).

In some embodiments, the decreased amount of C4b/C2b complexes in the sample of the later time point compared to the sample of the earlier time point indicates an amelioration of the disorder of the classical/lectin pathway and wherein the increased amount of C4b/C2b complexes in the sample of the later time point compared to the sample of the earlier time point indicates worsening of the disorder of the classical/lectin pathway.

In some embodiments, the decreased amount of C3b/Bb complexes in the sample of the later time point compared to the sample of the earlier time point indicates an amelioration of the disorder of the alternative pathway and/or the increased interaction of amount of C3b/Bb complexes in the sample of the later time point compared to the sample of the earlier time point indicates worsening of the disorder of the alternative pathway.

Another aspect of the invention relates to a method for determining whether a candidate compound is suitable for the treatment of a disease like a disorder of the complement system wherein the prediction of a suitable candidate compound is based on step (c), comprising the following steps:

(a) Incubating a sample of a subject with a candidate compound
(b) Detecting the presence of C3/C5 convertase complex comprising the steps:

(i) Determining the amount of C4b/C2b complexes in the sample and/or
(ii) Determining the amount of C3b/Bb complexes in the sample;

(c) Predicting whether a candidate compound is suitable for the treatment of a disorder of the complement system based on the result of step (b).

In some embodiments, the decreased amount of C4b/C2b complexes compared to a control may indicate that the candidate compound is suitable for the treatment of the disorder of the complement system. Accordingly, the decreased amount of C3b/Bb complexes compared to a control compound may indicate that the candidate compound is suitable for the treatment of the disorder of the complement system.

Another aspect of the invention refers to a kit comprising:

a) probes for detecting the complexes of C4b and C2b comprising:

- probe containing a moiety specifically binding C4b and a first signal generating moiety,
- probe containing a moiety specifically binding C2b and a second signal generating moiety,

b) probes for detecting complexes of C3b and Bb comprising:

- probe containing a moiety specifically binding C3b and a first signal generating moiety,
- probe containing a moiety specifically binding Bb and a second signal generating moiety.

In some embodiments, the analyte binding moiety (also termed "moiety specifically binding") and the signal generating moiety are part of the same molecule. In alternative embodiments, the analyte binding moiety and the signal generating moiety are different molecules.

A moiety specifically binding C4b, C2b, C3b or Bb may for example be an antibody and binding fragment thereof, a nanobody, a non-antibody protein scaffold protein (Vazquez-Lombardi R. et al.), aptamere, nucleotide based molecule and a small molecule.

In specific embodiments, the first and the second signal generating moieties build a FRET pair. In other embodiments, the first and the second signal generating moieties build an *in situ* proximity ligation pair.

## FIGURE LEGENDS

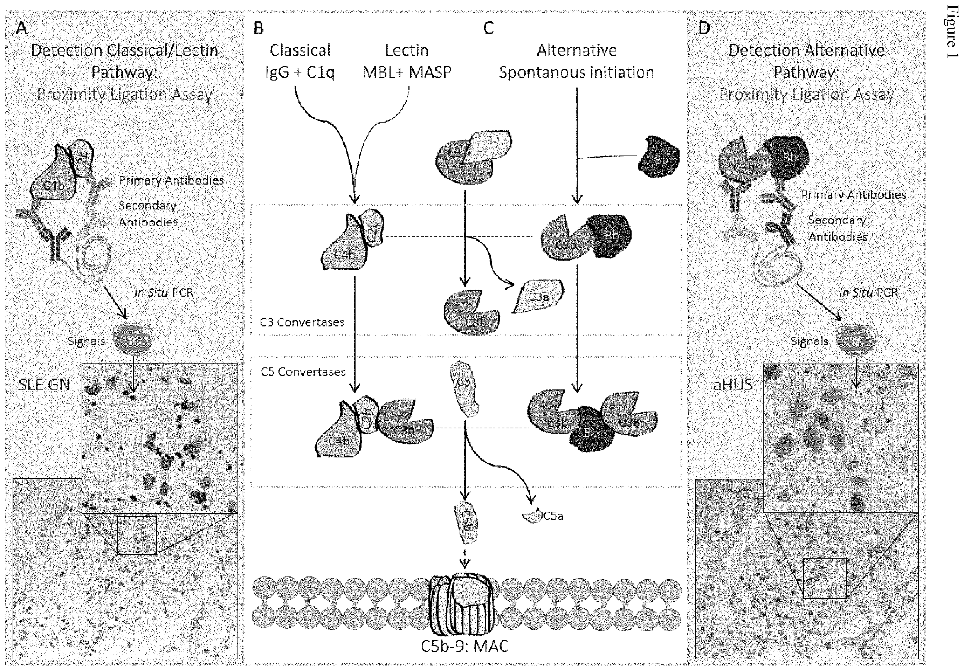

**Figure 1:** The complement cascade can be initiated by three pathways: the classical, lectin, and alternative pathway (B, C). Classical and lectin pathway activation results in assembly of C4b and C2b whereas the alternative pathway activation leads to assembly of C3b and Bb. Both C4bC2b and C3bBb are C3 convertases, which cleave surrounding C3, or after binding of another C3b molecule act as C5 convertases. The assembled complexes were detected by visualization of close proximity of their components by proximity ligation assay (A, D). Primary antibody binding was followed by the application of secondary antibodies, which are linked to oligonucleotides. The latter form rings enabling an *in situ* polymerase chain reaction with labeled probes resulting in a brown dot like signal for each C4b/C2 (A, bottom) and C3b/Bb (D, bottom) pair.

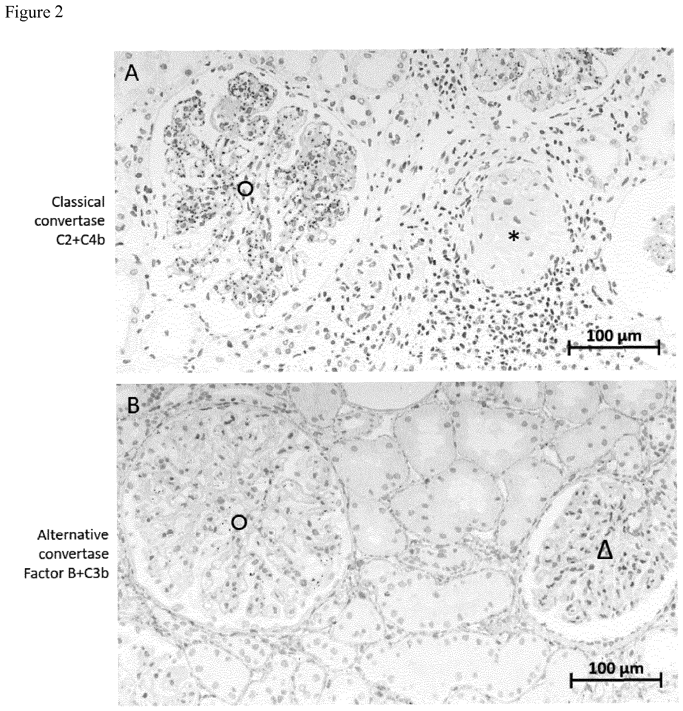

**Figure 2:** Exemplary depiction of glomeruli included and excluded from analysis.

A) Proximity ligation assay for classical/lectin pathway C3/C5-convertase in an SLE case. Note a preserved glomerulus with numerous signals included into the analysis (○) and a representative scarred glomerulus without signals (*) excluded from analysis.

B) Proximity ligation assay for alternative pathway C3/C5-convertase in an aHUS case. Note a preserved glomerulus with signals included into the analysis (○) and a representative collapsed glomerulus without signals (Δ) excluded from analysis.

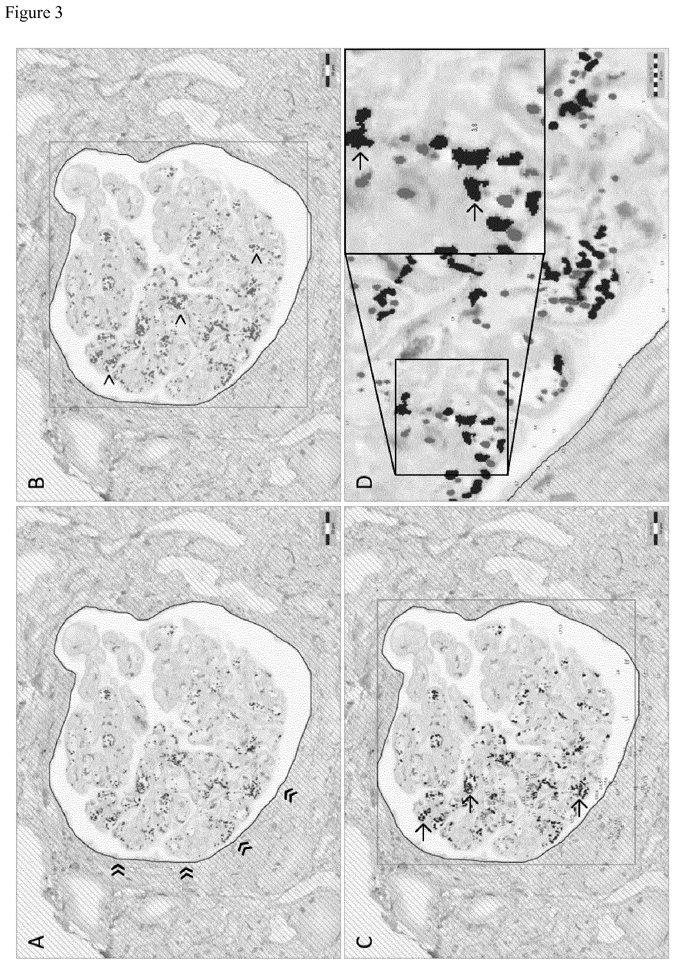

**Figure 3:** Overview of the computer-assisted quantification of signals in glomeruli.

A) Glomerulus of an aHUS case as region of interest (») for subsequent analysis. Note the abundant brown dot-like proximity ligation assay signals for the alternative pathway C3/C5-convertase inside the glomerular capillary lumens.

B) Detection of proximity ligation assay signals inside the defined region of interest. Note the green mark-up of the signals (>) according to the set parameters.

C), D) Automatized splitting of several confluent or overlapping signals into single signals (→). Note dark-green mark-up of the overlapping signals (→) and the number of estimated signals inside the respective area, subsequently rounded up, printed below the signals.

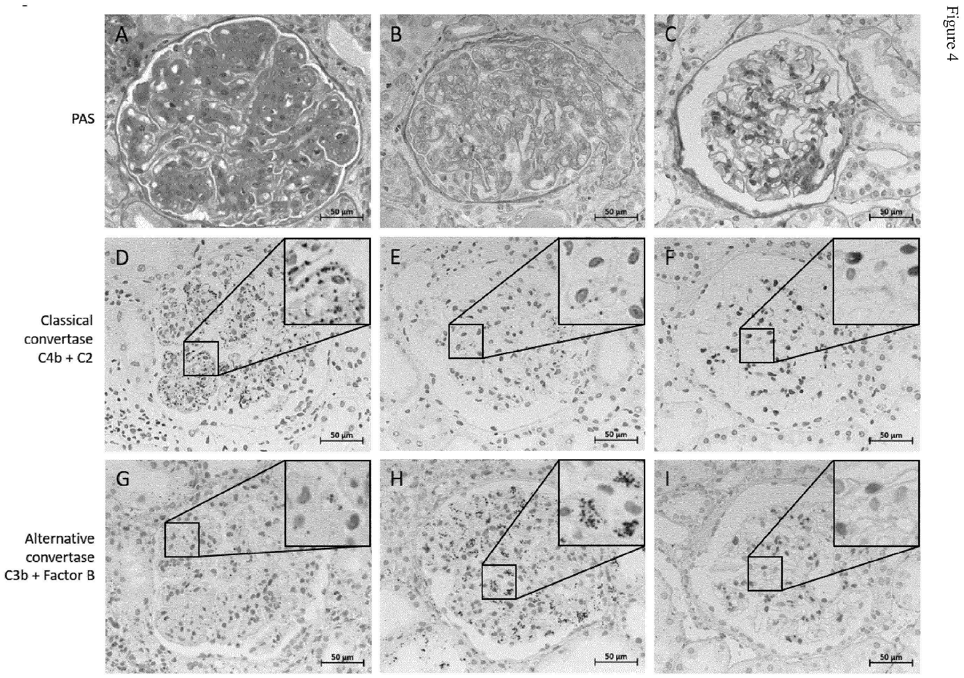

**Figure 4:** Proximity ligation assay for C4b/C2 and C3b/Factor B

A - C) PAS reaction of lupus nephritis (SLE GN), thrombotic microangiopathy due to atypical hemolytic syndrome (aHUS) and zero hour transplant biopsy as controls.

D- F) Proximity ligation assay for C4b/C2 of systemic lupus erythematosus (SLE), atypical hemolytic uremic syndrome (aHUS) and zero hour biopsies. Note brown dot-like PLA signals indicating assembly of the C3/C5 convertase of the classical/lectin pathway, most abundant in SLE (D) being located within the mesangium and along the peripheral glomerular capillary walls.

H - I) Proximity ligation assay for C3b/Factor B of systemic lupus erythematosus (SLE), atypical hemolytic uremic syndrome (aHUS) and zero hour biopsy. Note brown dot-like PLA signals indicating assembly of the C3/C5 convertase of the alternative pathway with most signals in aHUS (H) being located within the glomerular capillary lumens.

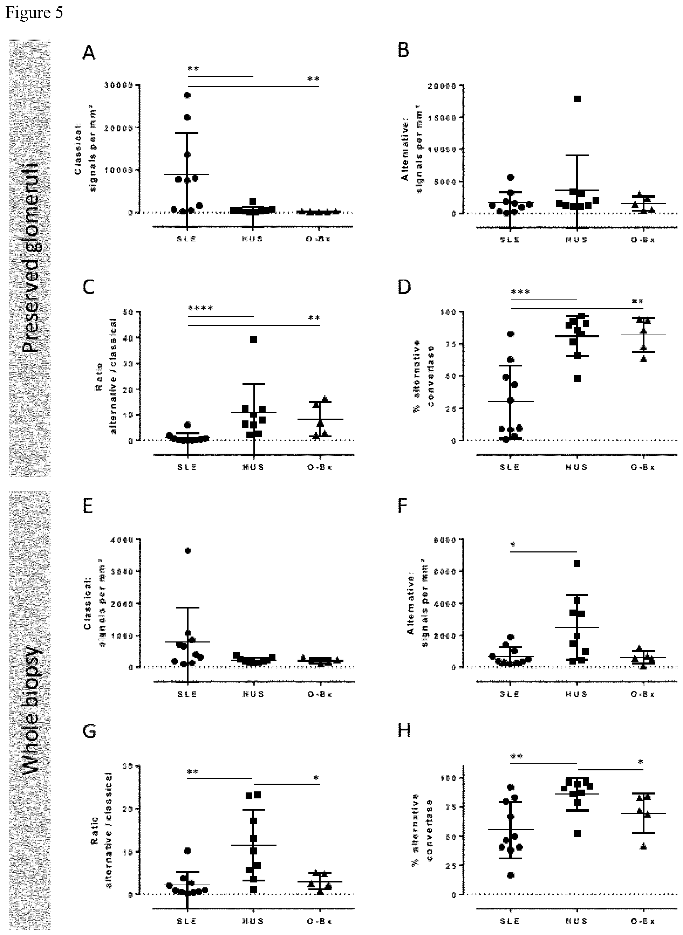

**Figure 5:** Results of the calculated signals in the preserved, presumably functional glomeruli only (A-D) and in whole biopsies (E-H). The classical convertases show significantly higher densities in SLE cases, as compared to HUS cases or zero hour transplant biopsies (O-Bx) taken as controls (A and E). In contrast, densities of the alternative convertase, as well as the alternative/classical pathway ratios were higher in HUS biopsies (B and F; C and G). The percentage of alternative pathway signals of all signals is lower in SLE cases compared to HUS cases and controls (D and H). Two-tailed Mann Whitney test was used to evaluate differences between the values (*=p<0.05; **=p<0.01; ***=p<0.001; ****=p<0.0001).

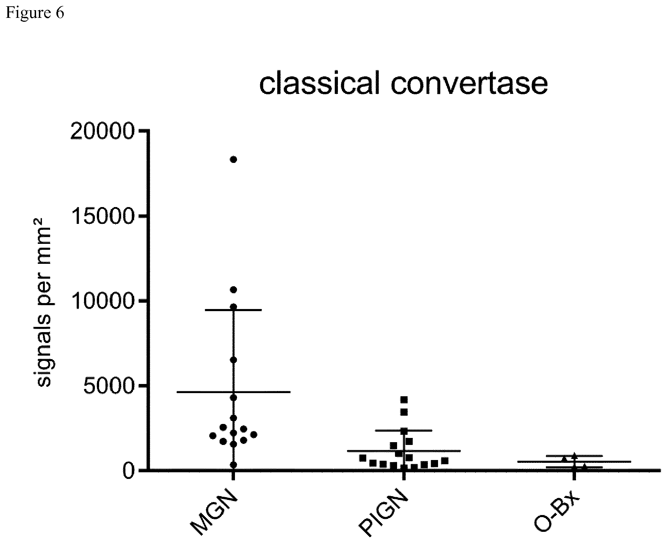

**Figure 6:** Results of proximity ligation assays for C4b/C2 in membranous nephropathy (MGN, n=15) and post-/parainfectious glomerulonephritis (PIGN, n=16). Nearly all cases of MGN show an enhanced signal density (average numbers of signals per mm2 glomerular area per case) for the assembly of the classical convertases compared to zero hour transplant biopsies (O-Bx) as normal controls. In contrast, only few cases of PIGN reveal an enhanced signal density. These preliminary results fit well into the hypotheses that in MGN subepithelial immune complexes initiate the classical complement pathway whereas in most cases of PIGN a direct activation of the alternative pathway is more likely.

## DETAILED DESCRIPTION OF THE INVENTION

Before the invention is described in detail with respect to some of its preferred embodiments, the following general definitions are provided.

The present invention as illustratively described in the following may suitably be practiced in the absence of any element or elements, limitation or limitations, not specifically disclosed herein.

The present invention will be described with respect to particular embodiments and with reference to certain figures but the invention is not limited thereto but only by the claims.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group which preferably consists only of these embodiments.

For the purposes of the present invention, the term "obtained" is considered to be a preferred embodiment of the term "obtainable". If hereinafter e.g. an antibody is defined to be obtainable from a specific source, this is also to be understood to disclose an antibody which is obtained from this source.

Where an indefinite or definite article is used when referring to a singular noun, e.g. "a", "an" or "the", this includes a plural of that noun unless something else is specifically stated. The terms "about" or "approximately" in the context of the present invention denote an interval of accuracy that the person skilled in the art will understand to still ensure the technical effect of the feature in question. The term typically indicates deviation from the indicated numerical value of $\pm 10\%$, and preferably of $\pm 5\%$.

Technical terms are used by their common sense. If a specific meaning is conveyed to certain terms, definitions of terms will be given in the following in the context of which the terms are used.

C3 convertase is a protein complex comprising several protein subunits that acts as serine protease converting the protein C3 to C3a and C3b fragments. In particular embodiments, the term C3 convertase denotes two different protein complexes that can be distinguished by the present invention: (i) the C3 convertase of the classical/lectin pathway that contains the two proteins C4b and C2b; (ii) the C3 convertase of the alternative pathway that contains Bb and one C3b subunit.

C5 convertase is a protein complex comprising several proteins that acts as serine protease converting the protein C5 to C5a and C5b fragments. In particular embodiments, the term C5 convertase denotes two different protein complexes that can be distinguished by the present invention: (i) the C5 convertase of the classical/lectin pathway that contains the two proteins C4b and C2b and C3b; (ii) the C5 convertase of the alternative pathway that contains the protein Bb and two C3b proteins.

C3/C5 convertase complex denotes protein complexes that contain at least the components of the C3 convertase, i.e. (i) the C3 convertase of the classical/lectin pathway that contains the two proteins C4b and C2b; (ii) the C3 convertase of the alternative pathway that contains Bb and one C3b subunit, and may further contain (i) C3b resulting in the C5 convertase of the classical/lectin pathway or (ii) a second C3b subunit resulting in the C5 convertase of the alternative pathway.

C3 denotes human complement protein 3.

C4 denotes human complement protein 4.

C5 denotes human complement protein 5.

C2b also (formerly) termed C2a as used herein refers to the dissociated protein which is capable of binding to C4b.

C4b denotes the cleavage fragment of the complement component C4.

C3b denotes the cleavage fragment of the complement component C3.

Bb denotes the cleaved fragment/cleavage product of complement factor B.

C4b, C2b, C3b and Bb may for example be detected by commercially available antibodies such as anti-C3b antibody (mouse, Abcam, Cambridge, UK), anti-Factor B antibody (ANTI-CFB antibody produced in rabbit, HPA001832-100UL, Sigma Aldrich, Merck Darmstadt, Germany) anti-C4b antiserum (1:500, rabbit, Abcam, Cambridge, UK) and anti-C2 antibody (E-7) (1:10, mouse, Santa Cruz, sc-373809, Dallas, USA)

The terms "convertase" and "convertase complex" are used interchangeable herein.

A first aspect of the invention refers to a method for classifying a disorder associated with the complement system, comprising the following steps:

    (a) providing a sample of a subject

    (b) detecting the presence of C3/C5 convertase complex comprising the steps:

        (i) determining the amount of C4b/C2b complexes in the sample and/or

        (ii) determining the amount of C3b/Bb complexes in the sample;

    (c) classifying the disorder associated with the complement system based on the result of step (b).

The subject may be a mammal, such as a rodent (e.g. a mouse or rat), cat, dog, leporid (e.g. rabbit) or a primate, such as a monkeys, human. Preferably, the subject is a mouse or a human. More preferably, the subject is a human. The subject may be a healthy or suffering from a disease associated with the complement system.

Determining the amount of C4b/C2b complexes in the sample and/or determining the amount of C3b/Bb complexes in the sample means detecting the complexes of C4b and C2b and detecting complexes of C3b with Bb.

An increased amount of C4b/C2B complexes or C3b/Bb complexes refers in particular to a higher number of C4b/C2b complexes or C3b/Bb complexes, in particular to a higher number of signal spots each representing in general one C4b/C2b complex or C3b/Bb complex; in some embodiments it may also refer increased signal spots or spots with increased signal intensity, that may represent a higher number of complexes compared to smaller signal spots or spots with less signal intensity which may represent a smaller number of complexes.

Vice versa, an decreased amount of C4b/C2B complexes or C3b/Bb complexes refers in particular to a lower number of C4b/C2b complexes or C3b/Bb complexes, in particular to a lower number of signal spots each representing in general one C4b/C2b complex or C3b/Bb complex; in some embodiments it may also refer to decreased signal spots or spots with decreased signal intensity, that may represent a lower number of complexes compared to larger signal spots or spots with higher signal intensity which may represent a higher number of complexes.

The skilled person understands that the with higher resolutions each spot may represent a single complex while in a lower resolution a spot, in particular an enlarged spot or a spot with higher intensity may represent several complexes. The skilled person can differentiate between these two scenarios; There may be settings in which both scenarios occur in one sampled.

"Determining the amount of C4b/C2b complexes" or "determining the amount of C3b/Bb complexes" means measuring a signal representative for the amount of C4b/C2b complexes or C3b/Bb complexes.

Aberration in the context of the present invention refers to an altered activity. Thus "aberration of the classical/lectin pathway" means "altered activity of the classical/lectin pathway". Accordingly "aberration of the alternative pathway" means "altered activity of the alternative pathway".

Classifying the disorder of the complement system allows a more detailed specification of the disorder, in particular the classification, also termed discrimination, whether the disorder is associated with an aberration of the classical/lectin pathway and/or the diagnosis whether the disorder is associated with an aberration of the alternative pathway. In preferred embodiments, the classification discriminates between a disorder associated with an aberration of the classical/lectin pathway and a disorder associated with an aberration of the alternative pathway.

The skilled person understands that the methods described herein are *in vitro,* in particular *in situ,* methods. Therefore, diagnostic methods practiced on the human or animal body are not encompassed by the present description.

In some embodiments, the increased amount of C4b/C2b complexes compared to a control is indicative for a disorder associated with the classical/lectin pathway. Accordingly, the increased amount of C4b/C2b complexes compared to a predetermined threshold is indicative for a disorder associated with the classical/lectin pathway.

The skilled person is aware of methods for determining a threshold for the interaction of C4b and C2b that indicates whether a disorder is associated with the classical/lectin pathway. The threshold may be determined in a study with an appropriate number of patients.

Exemplarily, the threshold may be determined by the mean value of 30 healthy persons plus two standard deviations.

In some embodiments, the increased amount of C3b/Bb complexes compared to a control is indicative for a disorder associated with the alternative pathway. In other embodiments, the increased amount of C3b/Bb complexes above a predetermined threshold is indicative for a disorder associated with the alternative pathway.

In specific embodiments, a value of the amount of C3b/Bb complexes divided by the amount of C4b/C2b complexes larger than 1 is indicative for a disorder associated with an aberration of the alternative pathway.

Preferably the value of the amount of C3b/Bb complexes divided by the amount of C4b/C2b complexes is a ratio larger than 2, more preferably larger than 3, even more preferably larger than 4, most preferably larger than 5, such as larger than 10.

In some embodiments, an increased value of the amount of C3b/Bb complexes divided by the amount of C4b/C2b complexes compared to a control is indicative for a disorder associated with an aberration of the alternative pathway.

The term "control" as used herein refers to a sample, such as a kidney biopsy of an individual which has no aberrations of the complement system, in particular not aberrations of the classical/lectin pathway and the alternative pathway. The skilled person understands that also an average of several such samples could is understood as control. The control may be for example a "zero-hour biopsy" of the same tissue as the sample to be tested. The term "zero-hour biopsy" as used herein refers to a biopsy that is taken from a (presumably healthy) transplanted kidney at the time of transplantation (time-point "zero-hour").

In some embodiments, a value of the amount of C4b/C2b complexes divided by the amount of C3b/Bb complexes larger than 1 is indicative for a disorder associated with an aberration of the classical/lectin pathway.

In step (b) the detection of the presence of the C3/C5 convertase complex may be determined by any method in the art useful the detection of in situ protein interaction, for example by in situ proximity ligation or by fluorescence resonance energy transfer (FRET). Thus, in some embodiments, in step (b) the detection of the presence of the C3/C5 convertase complex may be carried out by light microscopy, such as light microscopy is bright field microscopy or fluorescence microscopy.

FRET is based on the transfer of energy between two fluorophores, i.e. a donor and an acceptor, when these fluorophores are in close proximity. When the two fluorophores come close enough to each other, excitation of the donor by an energy source triggers an energy transfer towards the acceptor, which in turn emits specific fluorescence at a given wavelength. The skilled person is aware of FRET imaging techniques and how to adapt them (Jares-Erijman, Nature Biotechnology 21, 1387-1395 (2003)). In the present case, for the detection of the C3/C5 convertase of the classical/lectin pathway, a probe containing the analyte binding moiety and the signal generating moiety, such as an antibody targeting C4b linked to a FRET donor or FRET acceptor, may be used in combination with a further probe, such as an antibody targeting C3b and containing the respective counterpart of the FRET pair. Alternatively, probes, in which the analyte binding moiety and the signal generating moiety are separate molecules may be use. For example, the molecules containing the analyte binding moieties may be primary antibodies targeting C4b and C2b which may be used in combination with signal generating moieties, such as secondary antibodies containing an FRET donor, the other an FRET acceptor. Correspondingly, for the detection of the C3/C5 convertase of the alternative pathway, a probe, such as an antibody linked to an FRET donor or FRET acceptor would be used to detect C3b and a further probe, such as an antibody targeting Bb fused to the respective counterpart of the FRET pair. Alternatively, probes having separate molecules for detection and signal generation, such as primary antibodies targeting C3b and Bb in combination with secondary antibodies containing a FRET donor and a FRET acceptor, respectively, may be used.

The proximity ligation assay is commercially availbale, e.g. the Duolink® assay provided by SIGMA-Aldrich 8USA). The assay is described inter alia in Soderberg et al.. As described in the method section, for the detection of the C3/C5 convertase of the classical pathway, probe molecules containing the analyte binding moiety, such as primary antibodies targeting C4b and C2b may be used in combination with probe molecules containing the signal generating moiety, i.e. proximity ligation assay secondary antibodies. Correspondingly, for the detection of the C3/C5 convertase of the alternative pathway secondary proximity ligation antibodies targeting the primary antibodies targeting C3b and Bb may be used. In principle, alternatively, also probes containing both the analyte binding moiety that target the complex subunits (i.e. C4b, C2b, C3b and Bb) and the signal generating moiety containing the proximity ligation assay domains can be used.

In some embodiments, the measurement may be carried out in the whole biopsy sample or in intact glomeruli.

The term whole biopsy sample refers to the entire sample without any discrimination of substructures such as glomeruli.

The term "measurement is carried out in intact glomeruli" means that only glomeruli that are intact are measured. The term intact glomeruli" refers to glomeruli of normal appearance, i.e. not scarred, not sclerosed, not necrotic, and not collapsed.

The term "disorder associated with the complement system" refers to diseases due to aberrations of the complement system. In particular, to diseases that lead to an aberrant number of C3/C5 convertases of the classical pathway or to an aberrant number of C3/C5 convertases of the alternative pathway.

In some embodiments, the disorder associated with the complement system is selected from the group consisting of kidney disease, cancer, autoimmunity, infection, degeneration and neuropathy. The kidney disease may be selected from the group consisting of systemic lupus erythematodes (SLE), atypical hemolytic uremic syndrome (aHUS), Shiga-toxin producing E. coli hemolytic uremic syndrome (STEC-HUS), secondary hemolytic uremic syndrome (secondary HUS), thrombotic microangiopathy caused by atypical hemolytic uremic syndrome (aHUS), C3 glomerulopathy, cryoglobulinemia, glomerulonephritis and dense deposit disease.

The glomerulonephritis may be selected from the group IgA nephritis, ANCA-associated nephritis, C3 glomerulonephritis, dense deposit disease, membranoproliferative glomerulonephritis (MPGN), membranous nephropathy, post-/parainfectious glomerulonephritis.

In some embodiments, the disorder associated with an aberration of the classical/lectin pathway is selected from the group consisting of cryoglobulinemia, membranous nephropathy and SLE, preferably SLE.

In other embodiments, the disorder associated with an aberration of the alternative pathway is aHUS post-/parainfectious glomerulonephritis and C3 glomerulopathy.

Typically, the sample is selected from the group consisting of body fluid or tissue sample. In some embodiments, the body fluid is selected from the group consisting of urine, liquor, blood, plasma, serum, effusion and synovial fluid. Preferably, the tissue sample is a kidney tissue sample.

Another aspect of the invention refers to a method for determining whether a patient with a disorder associated with the complement system will be responsive to a modulator of the classical/lectin pathway or alternative pathway, comprising the following steps:

(a) providing a sample of the patient;
(b) detecting the presence of C3/C5 convertase complex comprising the steps:

(i) determining the amount of C4b/C2b complexes in the sample and/or
(ii) determining the amount of C3b/Bb complexes in the sample;

(c) predicting a whether the patient will be responsive to the modulator of the classical/lectin pathway or alternative pathway based on the result of step (b).

In preferred embodiments, the modulator is an inhibitor.

The term "modulator", in particular "inhibitor", includes small molecules, antibodies and binding fragments thereof, non-antibody protein scaffold proteins, aptameres and nucleotide based molecules.

In some embodiments, the inhibitor is selected from the group consisting of Eculizumab and Ravulizumab (C5 inhibitors, both Alexion); C1 inhibitors Berinert and Cinryze; soluble C5a peptide binding mAb (IFX-1; InflaRx); C5a receptor antagonist (Avacopan, Chemocentryx); OMS 721 also termed, narsoplimap, a MASP2 inhibitor (Omeros); Amy 101 (Amyndas); APL2 a C3 targeting peptide (Apellis); Danicopan (ACH-4471), a Factor D binding antagonist (Achillion); LNP023 a Factor B blocking compound (Novartis).

In some embodiments, the increased amount of C4b/C2b complexes indicates that the patient will be responsive to an inhibitor of the classical/lectin pathway and/or the increased amount of C3b/Bb complexes indicates that the patient will be responsive to an inhibitor of the alternative pathway.

Another aspect of the invention refers to a method for determining the disorder course of a patient with a disorder associated with the complement system, comprising the following steps:

(a) Providing samples of a subject of at least two time points of the disease course;
(b) Detecting the presence of C3/C5 convertase complex in the samples comprising the steps:

(i) Determining the amount of C4b/C2b complexes in the samples and/or
(ii) Determining the amount of C3b/Bb complexes in the samples;

(c) Determining the disorder course based on the result of step (b).

In some embodiments, the decreased amount of C4b/C2b complexes in the sample of the later time point compared to the sample of the earlier time point indicates an amelioration of the disorder of the classical/lectin pathway and wherein the increased amount of C4b/C2b complexes in the sample of the later time point compared to the sample of the earlier time point indicates worsening of the disorder of the classical/lectin pathway.

In some embodiments, the decreased amount of C3b/Bb complexes in the sample of the later time point compared to the sample of the earlier time point indicates an amelioration of the disorder of the alternative pathway and/or the increased amount of C3b/Bb complexes in the sample of the later time point compared to the sample of the earlier time point indicates worsening of the disorder of the alternative pathway.

Another aspect of the invention relates to a method for determining whether a candidate compound is suitable for the treatment of a disorder of the complement system wherein the prediction of a suitable candidate compound is based on step (c), comprising the following steps:

(a) Incubating a sample of a subject with a candidate compound
(b) Detecting the presence of C3/C5 convertase complex comprising the steps:

(i) Determining the amount of C4b/C2b complexes in the sample and/or
(ii) Determining the amount of C3b/Bb complexes in the sample;

(c) Predicting whether a candidate compound is suitable for the treatment of a disorder of the complement system based on the result of step (b).

In some embodiments, the decreased amount of C4b/C2b complexes compared to a control may indicate that the candidate compound is suitable for the treatment of the disorder of the complement system. Accordingly, the decreased amount of C3b/Bb complexes compared to a control compound may indicate that the candidate compound is suitable for the treatment of the disorder of the complement system.

Another aspect of the invention refers to a kit comprising:

a) probes for detecting the complexes of C4b and C2b comprising:

- probe containing a moiety specifically binding C4b and a first signal generating moiety,
- probe containing a moiety specifically binding C2b and a second signal generating moiety,

b) probes for detecting complexes of C3b and Bb comprising:

- probe containing a moiety specifically binding C3b and a first signal generating moiety,
- probe containing a moiety specifically binding Bb and a second signal generating moiety.

In some embodiments, the analyte binding moiety and the signal generating moiety are part of the same molecule.

In alternative embodiments, the analyte binding moiety and the signal generating moiety are different molecules.

In specific embodiments, the first and the second signal generating moieties build a FRET pair. In other embodiments, the first and the second signal generating moieties build an *in situ* proximity ligation pair.

## EXAMPLES

Ten kidney biopsies with lupus nephritis (SLE, class IV), nine cases with thrombotic microangiopathy (TMA) due to atypical hemolytic uremic syndrome, and five zero hour transplant biopsies (control) were analyzed. Preserved glomeruli were defined as regions of interest while scarred or collapsed glomeruli were excluded (Fig. S1 in the Supplementary Appendix). Proximity ligation signal densities (number of signals per glomerular area [$mm^2$]) were determined using image analysis software (hs analysis), and compared between the groups (Fig. S2 in the Supplementary Appendix).

As expected, SLE cases revealed higher densities of classical/lectin convertases within the glomerular mesangium and around capillary walls (median 7,685 signals/$mm^2$), as compared to TMA biopsies (median 393 signals/$mm^2$) and to control biopsies (median 207 signals/$mm^2$). In contrast, TMA cases showed a predominance of alternative convertases with most signals being located within the glomerular capillary lumens (median 3,032 signals/$mm^2$), as compared to SLE cases and control biopsies (median 1,329 signals/$mm^2$ and 1,418 signals/$mm^2$, respectively) (Fig. S3 and S4 in the Supplementary Appendix). Thus, we introduce the first methodological workflow for the visualization, differentiation and quantification of classical/lectin and alternative C3/C5 convertases directly within the tissue specimen. This new approach represents a tool to discriminate complement pathways in tissue and to show the dynamics of activation, enhancing diagnosis and potentially allowing future efficacy monitoring of individualized therapy.

Patients and Biopsies

From the archive of the pathology institute of the University Hospital Hamburg-Eppendorf (UKE), 10 cases of systemic lupus erythematodes nephritis class IV and 9 cases of atypical hemolytic uremic syndrome were selected. Five transplant zero hour biopsies were chosen as 1 control. The study was approved by the local ethics committee for Hamburg (Ethics Commission Hamburg, PV 5541). All work was carried out in accordance with the Declaration of Helsinki.

Methods

Immunohistochemistry and Proximity Ligation Assay

Slides (1$\mu$m) from paraffin embedded kidney biopsies were deparaffinized, pretreated and blocked using normal horse serum (Vector Industries S-2000). As pretreatment for C3b and Factor B detection Dako Target Retrieval Solution (S1699, Sigma-Aldrich, Darmstadt, Germany) was applied for 15 min at 120°C. Primary antibodies against C3b (1:1000, mouse) (Abcam, Cambridge, UK) and Factor B (1:100, rabbit) (Sigma Aldrich, Darmstadt, Germany) were applied at 4°C for 16 hours. For detection of the classical C3/C5 convertase (C4b and C2) the tissue sections were pretreated with protease for 30 min at 40°C (P8038, Sigma-Aldrich, Darmstadt, Germany). Afterwards C4b antiserum (1:500, rabbit, Abcam, Cambridge, UK) and C2 antiserum (1:10, mouse, Santa Cruz, Dallas, USA) were applied at 4°C for 16 hours. Next, proximity ligation assay secondary antibodies against mouse (Duolink® In Situ PLA Probe Anti-Mouse Minus, Sigma Aldrich, Darmstadt, Germany) and rabbit (Duolink® In Situ PLA Probe Anti-Rabbit Plus, Sigma Aldrich, Darmstadt, Germany) antibodies were added. According to protocol the brightfield detection system (Duolink® In Situ Detection Reagents Brightfield, Sigma Aldrich, Darmstadt, Germany) was applied to the slides.

The same primary antibodies were used on subsequent slides to visualize the deposition of C4b, C2, C3b and Factor B individually. For staining of C3 (1:3000, rabbit, polyclonal), C1q (1:1500, rabbit, polyclonal) (both DAKO, Eching, Germany) and IgG (1:7500, mouse, monoclonal) (Dianova, Hamburg, Germany), slides were pretreated with protease (15 min, 40°C) (P8038, Sigma-Aldrich, Darmstadt, Germany) and a standard APAAP-Protocol (monoclonal or polyclonal) was used. Detection was performed with the ZytoChem Plus AP Polymer System (POLAP-100, Zytomed, Berlin, Germany) using new fuchsin red chromogen and haematoxylin nuclear counterstain.

Quantification of proximity ligation assay signals

Densities of signals in glomeruli and in whole biopsies were analyzed using image analysis software (HSA KIT, HS Analysis GmbH, Karlsruhe, Germany). Scarred or collapsed glomeruli were excluded from glomerular analyses. With the software HSA KIT the PLA signals were detected, counted and measured using the Maximally Stable Extremal Regions (MSER) algorithm, which is described below. The following parameters were defined for this purpose: color ranges separating the foreground from the background of the signals within the biopsy as well as intervals for the intensity and for the size of the signals. Two categories of false positive signals occurred; intramembranous pseudo signals and intranuclear pseudo signals in cases with strong nuclear counterstaining. False positive intramembranous pseudo signals occurred only very rarely, so that they were negligible for the evaluation. In order to minimize the number of false positive intranuclear signals, a further parameter set was defined to determine the intensity and color values for negative signals. These negative signals are also detected via the MSER algorithm using an additional parameter set consisting of intensity and color values.

Negative signals and especially regions where positive and negative signals overlapped were excluded from the evaluation. A live overlay showing both the positive and negative signals allowed for easy parameter tuning until satisfactory values were found.

The MSER algorithm detects a number of features in an image. These MSERs are stable connected components that are chosen from a binary threshold representation of the image. The algorithm works as follows. A set of n equidistant threshold levels for the image signals $T = \{T_1 .. T_n\}$ is chosen. For every threshold level a pixel set $S_i = \{x : I(x) \leq T_i\}$ is generated from image $I(x)$ where $x = \begin{pmatrix} x_1 \\ x_2 \end{pmatrix}$ is a two-dimensional index. For every set $S_i$ connected regions $R_i \subset S_i$ are detected and arranged as a tree, $R_i$ is a child region of $R_i + 1$ if $R_i \subset R_{i+1}$. The goal is now to select regions of maximal stability, in the sense that they do not change over different threshold levels. For that the algorithm assumes that regions that have a similar size as their child regions also do not change much in shape. Every region is assigned an instability score

$$z(R_i) = \frac{|R_i - R_i + 1|}{|R_i|}$$

The regions with minimal instability score are selected. Optionally the score can be modified to exclude regions that are too small ($|R_i| < min$) or too big ($c > max$) in size:

$$z(R_i) = \begin{cases} \infty & \text{if } |R_i| < \min \text{ or } |R_i| > \max \\ \dfrac{|R_i - R_i + 1|}{|R_i|} & \text{otherwise} \end{cases}$$

Statistical analyses

Data were presented as mean $\pm$ standard deviation and median. A two-tailed Mann Whitney test was used to evaluate differences between the values. All statistical analyses were performed using R Software version 3.4.3 packages rcorr and ggplot2 (R Foundation for Statistical Computing, Vienna, Austria). Visualization of the statistical results was performed using the GraphPad Prism software (GraphPad Software, La Jolla California USA). P < 0.05 was considered statistically significant.

The invention also relates to the following embodiments:

1. Method for classification a disorder associated with the complement system, comprising the following steps:

(a) providing a sample of a subject;
(b) detecting the presence of C3/C5 convertase complex in the sample of the subject; amount of C4b/C2b complexes amount of C3b/Bb complexes;
(c) classifying the disorder associated with the complement system based on the result of step (b).

2. Method according to embodiment 1 wherein step b) comprises the steps:

(i) determining the amount of C4b/C2b complexes in the sample and/or
(ii) determining the amount of C3b/Bb complexes in the sample;

3. Method according to embodiment 1, wherein the classification discriminates between an increased activity of the classical/lectin pathway and an increased activity of the alternative pathway.

4. Method according to any one of the preceding embodiments, wherein an increase amount of C4b/C2b complexes compared to a control is indicative for a disorder associated with the classical/lectin pathway.

5. Method according to any one of the preceding embodiments, wherein the increased amount of C4b/C2b complexes compared to a predetermined threshold is indicative for a disorder associated with the classical/lectin pathway.

6. Method according to any one of the preceding embodiments, wherein the increased amount of C3b/Bb complexes compared to a control or is indicative for a disorder associated with the alternative pathway.

7. Method according to any one of the preceding embodiments, wherein the increased amount of C3b/Bb complexes above a predetermined threshold is indicative for a disorder associated with the alternative pathway.

8. Method according to any one of the preceding embodiments, wherein a value of the amount of C3b/ Bb complexes divided by the amount of /C2b complexes larger than 1 is indicative for a disorder associated with an aberration of the alternative pathway.

9. Method according to any one of the preceding embodiments, wherein an increased value of the amount of C3b/Bb divided by the amount of C4b/C2b compared to a control is indicative for a disorder associated with an aberration of the alternative pathway.

10. Method according to any one of the preceding embodiments, wherein a value of the amount of C4b/C2b divided by the amount of C3b/ Bb larger than 1 is indicative for a disorder associated with an aberration of the classical/lectin pathway.

11. Method according to any one of the preceding embodiments, wherein in step (b) the detection of the amount of the C3/C5 convertase complex is determined by *in situ* proximity ligation in the sample.

12. Method according to any one of the preceding embodiments, wherein in step (b) the detection of the presence of the C3/C5 convertase complex is carried out by fluorescence resonance energy transfer (FRET).

13. Method according to any of the preceding embodiments, wherein in step (b) the detection of the presence of the C3/C5 convertase complex is carried out by light microscopy.

14. Method according to embodiment 13, wherein the light microscopy is bright field microscopy or fluorescence microscopy.

15. Method according to any one of the preceding embodiments, wherein the measurement is carried out in the whole biopsy sample.

16. Method according to any one of the preceding embodiments, wherein the measurement is carried out in intact glomeruli.

17 Method according to any one of the preceding embodiments, wherein the disorder associated with the complement system is selected from the group consisting of kidney disease, cancer, autoimmunity, infection, degeneration and neuropathy.

18. Method according to embodiment 17, wherein the kidney disease is selected from the group consisting of systemic lupus erythematodes (SLE), thrombotic microangiopathy caused by atypical hemolytic uremic syndrome (aHUS), Shiga-toxin producing *E. coli* hemolytic uremic syndrome (STEC-HUS), secondary hemolytic uremic syndrome (secondary HUS), C3 glomerulopathy, cryoglobulinemia, C3 glomerulonephritis, dense deposit disease, post-/parainfectious glomerulonephritis, IgA nephropathy, cryoglobulinemia, and membranoproliferative glomerulonephritis (MPGN).

19. Method according to any one of the preceding embodiments, wherein the disorder associated with an aberration of the classical/lectin pathway is selected from the group consisting of cryoglobulinemia, membranous nephropathy and SLE, preferably SLE.

20. Method according to any one of the preceding embodiments, wherein the disorder associated with an aberration of the alternative pathway is aHUS, post-/parainfectious glomerulonephritis and C3 glomerulopathy.

21. Method according to any one of the proceeding embodiments, wherein the sample is selected from the group consisting of body fluid or tissue sample.

22. Method according to embodiment 21, wherein the body fluid is selected from the group consisting of urine, liquor, blood, plasma, serum, effusion and synovial fluid.

23. Method according to any one of the proceeding embodiments, wherein the tissue sample is a kidney tissue sample.

24. Method for determining whether a patient with a disorder associated with the complement system will be responsive to a modulator of the classical/lectin pathway or alternative pathway, comprising the following steps:

(a) Providing a sample of the patient;
(b) Detecting the presence of C3/C5 convertase in the sample of the patient:
amount of C4b/C2b complexes (c) Predicting a whether the patient will be responsive to the inhibitor of the classical/lectin pathway or alternative pathway based on the result of step (b).

25. Method according to embodiment 24, wherein step b) comprises the steps:

(i) determining the amount of C4b/C2b complexes in the sample and/or

(ii) determining the amount of C3b/Bb complexes in the sample;

26. Method according to embodiment 24 or 25, wherein the modulator is an inhibitor.

27. Method according to embodiment 25, wherein an increased amount of C4b /C2b complexes indicates that the patient will be responsive to an inhibitor of the classical/lectin pathway and/or the increased amount of C3b/Bb complexes indicates that the patient will be responsive to an inhibitor of the alternative pathway.

28. Method for determining the disorder course of a patient with a disorder associated with the complement system, comprising the following steps:

(a) Providing samples of a subject of at least two time points of the disease course;
(b) Detecting the presence of C3/C5 convertase complex in the sample of the subject;
(c) Determining the disorder course based on the result of step (b).

29. Method according to embodiment 28, wherein step b) comprises the steps:

(i) Determining the amount of C4b/C2b complexes in the samples and/or
(ii) Determining the interaction of C3b with Bb in the samples;

30. Method according to embodiment 29, wherein the decreased amount of C4b/ C2b complexes in the sample of the later time point compared to the sample of the earlier time point indicates an amelioration of the disorder of the classical/lectin pathway and wherein the increased amount of C4b/C2b complexes in the sample of the later time point compared to the sample of the earlier time point indicates worsening of the disorder of the classical/lectin pathway.

31. Method according to embodiment 29 or 30, wherein the decreased amount of C3b/Bb complexes in the sample of the later time point compared to the sample of the earlier time point indicates an amelioration of the disorder of the alternative pathway and/or the increased amount of C3b/Bb complexes in the sample of the later time point compared to the sample of the earlier time point indicates worsening of the disorder of the alternative pathway.

32. Method for determining whether a candidate compound is suitable for the treatment of a disorder of the complement system wherein the prediction of a suitable candidate compound is based on step (c), comprising the following steps:

(a) Incubating a sample of a subject with a candidate compound
(b) Detecting the presence of C3/C5 convertase complex in the sample of step (a);
(c) Predicting whether a candidate compound is suitable for the treatment of a disorder of the complement system based on the result of step (b).

33. Method of embodiment 32, wherein step (b) comprises the following steps:

(i) Determining the amount of C4b/C2b complexes in the sample and/or
(ii) Determining the amount of C3b/ Bb complexes in the sample;

34. Method according to embodiment 32 or 33, wherein the decreased amount of C4b/C2b complexes compared to a control indicates that the candidate compound is suitable for the treatment of the disorder of the complement system.

35. Method according to embodiment 32 to 34, wherein the decreased amount of C3b/Bb compared to a control compound indicates that the candidate compound is suitable for the treatment of the disorder of the complement system.

36. Kit comprising:

a) probes for detecting the complexes of C4b and C2b comprising:

- probe containing an analyte binding moiety specifically binding C4b and a first signal generating moiety,

- probe containing an analyte binding moiety specifically binding C2b and a second signal generating moiety,

b) probes for detecting complexes of C3b and Bb comprising:

- probe containing an analyte binding moiety specifically binding C3b and a first signal generating moiety,
- probe containing an analyte binding moiety specifically binding Bb and a second signal generating moiety.

37. Kit according to embodiment 36, wherein the analyte binding moiety and the signal generating moiety are part of the same molecule.

38. Kit according to embodiment 36, wherein the analyte binding moiety and the signal generating moiety are different molecules.

39. Kit according to embodiments 36 to 38, wherein the first and the second signal generating moieties build a FRET pair.

40. Kit according to embodiments 36 to 38, wherein the first and the second signal generating moieties build an *in situ* proximity ligation pair.

## REFERENCES

Thurman et al. Complement in kidney disease: core curriculum 2015. Am J Kidney Dis 2015;65:156-68.

Zipfel et al. The role of complement in C3 glomerulopathy. Mol Immunol 2015;67:21-30. Reddy et al. Breaking down the complement system: a review and update on novel therapies. Curr Opin Nephrol Hypertens 2017;26:123-8.

Ricklin D et al. The renaissance of complement therapeutics. Nat Rev Nephrol 2018;14:26-47.

Soderberg et al. Direct observation of individual endogenous protein complexes in situ by proximity ligation. (Nat Methods, 3, 995-1000(2006))

Vazquez-Lombardi et al. Challenges and opportunities for non-antibody scaffold drugs. Drug Discovery Today 2015;20(10):1271-1283.

## Claims

1. Method for classification a disorder associated with the complement system, comprising the following steps:

   (a) providing a sample of a subject;
   (b) detecting the presence of C3/C5 convertase complex in the sample of the subject;
   (c) classifying the disorder associated with the complement system based on the result of step (b).

2. Method according to claim 1 wherein step b) comprises the steps:

   (i) determining the amount of C4b/C2b complexes in the sample and/or
   (ii) determining the amount of C3b/Bb complexes in the sample;

3. Method according to any one of the preceding claims, wherein an increase amount of C4b/C2b complexes compared to a control is indicative for a disorder associated with the classical/lectin pathway.

4. Method according to any one of the preceding claims, wherein the increased amount of C4b/C2b complexes compared to a predetermined threshold is indicative for a disorder associated with the classical/lectin pathway.

5. Method according to any one of the preceding claims, wherein in step (b) the detection of the amount of the C3/C5 convertase complex is determined by *in situ* proximity ligation in the sample.

**6.** Method according to any one of the proceeding embodiments, wherein the sample is selected from the group consisting of body fluid or tissue sample, wherein the sample is preferably a kidney tissue sample.

**7.** Method according to any one of the preceding claims, wherein the disorder associated with the complement system is selected from the group consisting of kidney disease, cancer, autoimmunity, infection, degeneration and neuropathy.

**8.** Method according to claim 7, wherein the kidney disease is selected from the group consisting of systemic lupus erythematodes (SLE), thrombotic microangiopathy caused by atypical hemolytic uremic syndrome (aHUS), Shiga-toxin producing *E. coli* hemolytic uremic syndrome (STEC-HUS), secondary hemolytic uremic syndrome (secondary HUS), C3 glomerulopathy, cryoglobulinemia, C3 glomerulonephritis, dense deposit disease, post-/parainfectious glomerulonephritis, IgA nephropathy, cryoglobulinemia, and membranoproliferative glomerulonephritis (MPGN).

**9.** Method according to any one of the preceding claims, wherein the disorder associated with an aberration of the classical/lectin pathway is selected from the group consisting of cryoglobulinemia, membranous nephropathy and SLE, preferably SLE and wherein the disorder associated with an aberration of the alternative pathway is a HUS, post-/parainfectious glomerulonephritis and C3 glomerulopathy.

**10.** Method according to any one of the proceeding claims, wherein the sample is selected from the group consisting of body fluid or tissue sample.

**11.** Method according to any one of the proceeding claims, wherein the tissue sample is a kidney tissue sample.

**12.** Method for determining whether a patient with a disorder associated with the complement system will be responsive to a modulator of the classical/lectin pathway or alternative pathway, comprising the following steps:

(a) Providing a sample of the patient;
(b) Detecting the presence of C3/C5 convertase in the sample of the patient:
amount of C4b/C2b complexes (c) Predicting a whether the patient will be responsive to the inhibitor of the classical/lectin pathway or alternative pathway based on the result of step (b).

**13.** Method for determining the disorder course of a patient with a disorder associated with the complement system, comprising the following steps:

(a) Providing samples of a subject of at least two time points of the disease course;
(b) Detecting the presence of C3/C5 convertase complex in the sample of the subject;
(c) Determining the disorder course based on the result of step (b).

**14.** Method for determining whether a candidate compound is suitable for the treatment of a disorder of the complement system wherein the prediction of a suitable candidate compound is based on step (c), comprising the following steps:

(a) Incubating a sample of a subject with a candidate compound
(b) Detecting the presence of C3/C5 convertase complex in the sample of step (a);
(c) Predicting whether a candidate compound is suitable for the treatment of a disorder of the complement system based on the result of step (b).

**15.** Kit comprising:

a) probes for detecting the complexes of C4b and C2b comprising:

- probe containing an analyte binding moiety specifically binding C4b and a first signal generating moiety,
- probe containing an analyte binding moiety specifically binding C2b and a second signal generating moiety,

b) probes for detecting complexes of C3b and Bb comprising:

- probe containing an analyte binding moiety specifically binding C3b and a first signal generating moiety,
- probe containing an analyte binding moiety specifically binding Bb and a second signal generating moiety.

Figure 1

Figure 2

Classical
convertase
C2+C4b

Alternative
convertase
Factor B+C3b

Figure 3

Figure 4

Figure 5

Figure 6

classical convertase

Europäisches
Patentamt
European
Patent Office
Office européen
des brevets

## EUROPEAN SEARCH REPORT

Application Number

EP 19 18 9415

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 03/068150 A2 (JOSLIN DIABETES CENTER INC [US]; BENOIST CHRISTOPHE O [US] ET AL.) 21 August 2003 (2003-08-21) | 1,2,6,7,10 | INV.<br>A61K39/395<br>C12Q1/6804<br>C12Q1/6862 |
| Y | * Claim 1, claim 2, p65 line 22-28, p65 line 10-19, p68 line 8-10, p2 line 27-28, * | 5 | |
| X | WO 2015/070041 A1 (ICAHN SCHOOL MED MOUNT SINAI [US]) 14 May 2015 (2015-05-14)<br>* Claim 1 (p54), Claim 2 (p54), Summary * | 13 | |
| X | WO 2019/099950 A1 (GOETZL EDWARD J [US]) 23 May 2019 (2019-05-23)<br>* Claim 1, Claim 7, Claim 16 * | 1,2 | |
| X | WO 2018/081400 A1 (ALEXION PHARMA INC [US]) 3 May 2018 (2018-05-03)<br>* p60 claims 1-4 * | 12,14 | |
| Y | WO 2011/123044 A1 (ANAMAR AB [SE]; BLOM ANNA [SE] ET AL.) 6 October 2011 (2011-10-06)<br>* claim 14 * | 15 | TECHNICAL FIELDS SEARCHED (IPC)<br><br>C07K<br>A61K<br>C12Q |
| Y | WO 2014/055835 A1 (NOVELMED THERAPEUTICS INC [US]) 10 April 2014 (2014-04-10)<br>* claim 26, 33 and 35 * | 15 | |
| Y | WO 2004/112572 A2 (UNIV PITTSBURGH [US]; AHEARN JOSEPH M [US]; MANZI SUSAN M [US]) 29 December 2004 (2004-12-29)<br>* claim 26 * | 15 | |

-/--

The present search report has been drawn up for all claims

| Place of search | Date of completion of the search | Examiner |
|---|---|---|
| Munich | 9 January 2020 | Hennard, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 19 18 9415

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SODERBERG O ET AL: "Characterizing proteins and their interactions in cells and tissues using the in situ proximity ligation assay", METHODS, ACADEMIC PRESS, NL, vol. 45, no. 3, 1 July 2008 (2008-07-01), pages 227-232, XP023437838, ISSN: 1046-2023, DOI: 10.1016/J.YMETH.2008.06.014 [retrieved on 2008-07-11] * p228 whole left column and second paragraph on right column * | 5 | |
| X | MATTHEW C. PICKERING ET AL: "C3 glomerulopathy: consensus report", KIDNEY INTERNATIONAL, vol. 84, no. 6, 1 December 2013 (2013-12-01), pages 1079-1089, XP55646662, LONDON, GB ISSN: 0085-2538, DOI: 10.1038/ki.2013.377 * [p1085 fourth paragraph on the right column], abstract * | 1,2,7-9 | |

TECHNICAL FIELDS
SEARCHED (IPC)

The present search report has been drawn up for all claims

| Place of search | Date of completion of the search | Examiner |
|---|---|---|
| Munich | 9 January 2020 | Hennard, Christophe |

EPO FORM 1503 03.82 (P04C01)

## ANNEX TO THE EUROPEAN SEARCH REPORT
## ON EUROPEAN PATENT APPLICATION NO.

EP 19 18 9415

This annex lists the patent family members relating to the patent documents cited in the above-mentioned European search report.
The members are as contained in the European Patent Office EDP file on
The European Patent Office is in no way liable for these particulars which are merely given for the purpose of information.

09-01-2020

| Patent document cited in search report | | Publication date | Patent family member(s) | | Publication date |
|---|---|---|---|---|---|
| WO 03068150 | A2 | 21-08-2003 | AU 2003209049 A1<br>WO 03068150 A2 | | 04-09-2003<br>21-08-2003 |
| WO 2015070041 | A1 | 14-05-2015 | NONE | | |
| WO 2019099950 | A1 | 23-05-2019 | NONE | | |
| WO 2018081400 | A1 | 03-05-2018 | EP 3532845 A1<br>WO 2018081400 A1 | | 04-09-2019<br>03-05-2018 |
| WO 2011123044 | A1 | 06-10-2011 | AU 2011233753 A1<br>EP 2553465 A1<br>JP 2013524213 A<br>US 2013084584 A1<br>WO 2011123044 A1 | | 13-09-2012<br>06-02-2013<br>17-06-2013<br>04-04-2013<br>06-10-2011 |
| WO 2014055835 | A1 | 10-04-2014 | AU 2013326932 A1<br>AU 2019213295 A1<br>CA 2887050 A1<br>CN 104870474 A<br>EP 2904014 A1<br>JP 2015532303 A<br>US 2015315268 A1<br>US 2016122419 A1<br>US 2019031747 A1<br>WO 2014055835 A1 | | 14-05-2015<br>29-08-2019<br>10-04-2014<br>26-08-2015<br>12-08-2015<br>09-11-2015<br>05-11-2015<br>05-05-2016<br>31-01-2019<br>10-04-2014 |
| WO 2004112572 | A2 | 29-12-2004 | AT 465275 T<br>AU 2004249155 A1<br>CA 2529045 A1<br>DK 1635692 T3<br>EP 1635692 A2<br>EP 2194383 A1<br>ES 2342291 T3<br>JP 2007501948 A<br>JP 2010164581 A<br>KR 20060015646 A<br>US 2005042602 A1<br>WO 2004112572 A2<br>ZA 200510325 B | | 15-05-2010<br>29-12-2004<br>29-12-2004<br>09-08-2010<br>22-03-2006<br>09-06-2010<br>05-07-2010<br>01-02-2007<br>29-07-2010<br>17-02-2006<br>24-02-2005<br>29-12-2004<br>30-04-2008 |

EPO FORM P0459

For more details about this annex : see Official Journal of the European Patent Office, No. 12/82

## REFERENCES CITED IN THE DESCRIPTION

**Non-patent literature cited in the description**

- **JARES-ERIJMAN.** *Nature Biotechnology,* 2003, vol. 21, 1387-1395
- **THURMAN et al.** Complement in kidney disease: core curriculum 2015. *Am J Kidney Dis,* 2015, vol. 65, 156-68
- **ZIPFEL et al.** The role of complement in C3 glomerulopathy. *Mol Immunol,* 2015, vol. 67, 21-30
- **REDDY et al.** Breaking down the complement system: a review and update on novel therapies. *Curr Opin Nephrol Hypertens,* 2017, vol. 26, 123-8
- **RICKLIN D et al.** The renaissance of complement therapeutics. *Nat Rev Nephrol,* 2018, vol. 14, 26-47
- **SODERBERG et al.** Direct observation of individual endogenous protein complexes in situ by proximity ligation. *Nat Methods,* 2006, vol. 3, 995-1000
- **VAZQUEZ-LOMBARDI et al.** Challenges and opportunities for non-antibody scaffold drugs. *Drug Discovery Today,* 2015, vol. 20 (10), 1271-1283